# EUROPEAN PATENT SPECIFICATION

(11) **EP 3 922 714 B1**
(45) Date of publication and mention of the grant of the patent: **01.04.2026**
(21) Application number: 20179392.4
(22) Date of filing: 10.06.2020
(51) Int. Cl.: C12N 5/071, G01N 33/50, A61F 2/10, A61P 31/12

(54) **USE OF 3D SKIN MODEL FOR ASSESSING TREATMENT OF SARS-CORONAVIRUS SKIN INFECTION**
VERWENDUNG EINES 3D-HAUTMODELLS ZUR BEURTEILUNG DER BEHANDLUNG VON SARS-CORONAVIRUS-HAUTINFEKTION
UTILISATION D'UN MODÈLE DE PEAU EN 3D POUR ÉVALUER LE TRAITEMENT D'UNE INFECTION SARS-CORONAVIRUS DE LA PEAU

(43) Date of publication of application: 15.12.2021
(73) Proprietor: PKDERM, 06600 Antibes (FR)
(72) Inventor: OSMAN-PONCHET, Hanan, 06600 Antibes (FR)
(74) Representative: Axe PI

(56) References cited:
- US-A1- 2011 300 530
- WAMBIER CARLOS GUSTAVO ET AL: "Severe acute respiratory syndrome coronavirus 2 (SARS-CoV-2) infection is likely to be androgen mediated", JOURNAL OF THE AMERICAN ACADEMY OF DERMATOLOGY, MOSBY, INC, US, vol. 83, no. 1, 10 April 2020 (2020-04-10), pages 308 - 309, XP086181548, ISSN: 0190-9622, [retrieved on 20200410], DOI: 10.1016/J.JAAD.2020.04.032
- CRIADO PAULO RICARDO ET AL: "Are the cutaneous manifestations during or due to SARS-CoV-2 infection/COVID-19 frequent or not? Revision of possible pathophysiologic mechanisms", INFLAMMATION RESEARCH, BIRKHAEUSER VERLAG, BASEL, CH, vol. 69, no. 8, 2 June 2020 (2020-06-02), pages 745 - 756, XP037178923, ISSN: 1023-3830, [retrieved on 20200602], DOI: 10.1007/S00011-020-01370-W
- XIAOTONG XUE ET AL: "High Expression of ACE2 on Keratinocytes Reveals Skin as a Potential Target for SARS-CoV-2", JOURNAL OF INVESTIGATIVE DERMATOLOGY, 1 May 2020 (2020-05-01), NL, XP055737240, ISSN: 0022-202X, DOI: 10.1016/j.jid.2020.05.087
- SUNGNAK WARADON ET AL: "SARS-CoV-2 entry factors are highly expressed in nasal epithelial cells together with innate immune genes", NATURE MEDICINE, NATURE PUB. CO, NEW YORK, vol. 26, no. 5, 23 April 2020 (2020-04-23), pages 681 - 687, XP037113619, ISSN: 1078-8956, [retrieved on 20200423], DOI: 10.1038/S41591-020-0868-6
- BART DE WEVER ET AL: "Human Skin Models for Research Applications in Pharmacology and Toxicology: Introducing NativeSkin , the "Missing Link" Bridging Cell Culture and/or Reconstructed Skin Models and Human Clinical Testing", APPLIED IN VITRO TOXICOLOGY, vol. 1, no. 1, 1 March 2015 (2015-03-01), US, pages 26 - 32, XP055538973, ISSN: 2332-1512, DOI: 10.1089/aivt.2014.0010

## Description

The present invention is relating to the medical domain and regards to the treatment of SARS-coronavirus skin infection.

More particularly, the instant invention is relative to an *in vitro* assay for assessing treatment of SARS-Coronavirus skin infection, by analyzing the target receptors modulation. By modulation, it is meant increase or decrease of target receptors gene expression variation. Hence, detection and/or activation of specific SARS-coronavirus target receptors is an indication of skin cells infection and its treatment.

Indeed, with the hypothesis that skin may be a potential door to entry in the body of SARS-coronaviruses and more recently the SARS-coronavirus 2 (SARS-CoV-2 also called COVID-19) and that androgen receptors (AR) may play a role in the severity of the COVID-19 patients. Therefore, blockade of AR in the skin may prevent skin infection of SARS-CoV-2.

The recent emergence of the novel, pathogenic SARS-CoV-2 in China and its rapid national and international spread pose a global health emergency. Cell entry of coronaviruses depends on binding of the viral spike (S) proteins to the Angiotensin Converting Enzyme 2 (ACE2) receptors and on S protein priming by host cell protease TMPRSS2 (Hoffmann et al., 2020). High expression of ACE2 in skin tissues, in particular keratinocytes, rendering skin as potential target for SARS-CoV-2 infection is discussed in Xiaotong Xue et al. in journal of investigative dermatology, 1 May 2020. The role of the proteins S, ACE2 and TMPRSS2 is especially described in Criado Paulo Ricardo et al. in inflammation research, 2 June 2020. It discloses a link between actions of SARS-CoV-2 and human skin. The possibility of direct entry of SARS-CoV-2 via receptor ACE2 and protease TMPRSS2 in the human endothelial cell in dermal blood vessels is discussed.Generally, ACE2 may be expressed in different parts of the human body including the lung, small intestine, colon, duodenum, kidney, testis, gallbladder, heart, esophagus, and urinary bladder, rendering these organs a potential target for the SARS-CoV-2. Interestingly, ACE2 is also present in the basal cell layer of the epidermis continuing to the basal cell layer of the hair follicles, the smooth muscle cells that enclose the sebaceous glands, and the eccrine glands. It is notably discussed in Sungnak Waradon et al. in nature medicine, 23 April 2020, which discloses the expression levels of the SARS-CoV-2 entry factors ACE2 and TMPRSS2 in nasal epithelial cells. ACE2 and TMPRSS2 expression in developmental data sets from fetal tissues, including liver, thymus, skin, bone marrow, yolk sac and lung is also assessed.

It is hypothesized that androgen receptors (AR) play a role in the severity of the COVID-19 patients. This hypothesis was based on the disproportionate prevalence of severe COVID-19 among children and adult females compared to adult males. Besides, the ACE2 activity have shown to decrease with the reduction of the androgen hormones. However, this theory is not yet proven by robust data.

The theory that androgen mediates SARS-CoV-2 infection is discussed in Wambier Carlos Gustavo et al. in J. of the American academy of dermatology, 10 April 2020 : TMPRSS2 cleaves ACE2 for augmented viral entry. Androgen receptor activity has been considered a requirement for the transcription of the TMPRSS2 gene because no other known TMPRSS2 gene promoter has been described in humans to date.

The SARS-CoV-2 is a highly contagious disease and is transmitted through respiratory droplets of infected person in either a direct transmission (close contact with infected person) or indirect transmission. Finally, hidden transmission from asymptomatic carriers may occurs.

There is a broad spectrum of manifestations in COVID-19 patients: fever, dry cough, dyspnea and myalgia with headache, nausea and diarrhea being less frequent manifestations.

On the other hand, COVID-19 can affect different organ systems, including the skin (Rodriguez-Jiménez et al., 2020). Regarding the cutaneous manifestation of COVID-19, a recent Italian study of 88 COVID-19 patients showed that 20.4% of the patients demonstrated cutaneous manifestation in the form of erythematous rash, urticaria, and chickenpox-like vesicles mainly in the trunk with little or no itching. Furthermore, the authors reported that those skin lesions were not correlated with disease severity. COVID-19 patients may present with urticarial eruption without any respiratory symptoms (cough or fever).

Generally, skin is considered a part of the immune system as it acts as a shield against different environmental stimuli. In this setting, compromising the integrity of this barrier due to frequent cleansing and/or long-term use of personal protective equipment (PPE), or pre-existing infections or diseases of skin, wounds, and burns may facilitate the invasion by micro-organisms. Therefore, people may be at higher risk of SARS-CoV-2 due to the loss of the skin integrity.

Commercially available skin models are currently used for evaluating chemical substances, drugs, and cosmetics. These models have been evaluated and have demonstrated similarities to native human tissues in terms of morphology, lipid composition and biochemical markers. Thus, Bart De Wever et al. in applied in vitro toxicology, 1 March 2015, describes a 3D human native skin model comprising a keratinocyte layer for assessing topically applied substances. These models have been identified as useful tools for the testing of phototoxicity, corrosivity and irritancy as disclosed by F. Netzlaff et al. in Eur J Pharm Biopharm. 2005 Jul;60(2):167-78.

Similarly, PCT application WO2014132063 describes a three-dimensional (3-D) model comprising a scaffold and autologous skin cells and methods of predicting immunogenicity and hypersensitivity or allergic/adverse immune reactions to potential therapeutic compounds, cosmetics and chemical sensitizers using the 3-D model of skin cells. In the same way, US patent application US2011/300530 discloses a human skin model comprising a keratinocyte layer for examining viral infections and active ingredients with anti-viral action. The system is used to assess viral infection wherein infection, i.e. viral activation is verified by qRTPCR of virus-relevant gene expression.

There is therefore a need for a simple, robust, cost-effective, accurate assay for evaluating efficacy treatment of novel compounds, known drugs or any other topical treatment (cosmetic, botanical extract, or chemical product) onto skin after SARS-coronavirus infection.

The present invention provides a technical solution to the above-mentioned problems and provides a method for assessing treatment of skin infection by SARS-coronavirus and more preferably by SARS-CoV-2.

Hence the instant invention regards to an in vitro assay for assessing treatment of SARS-Coronavirus skin infection, preferably SARS-CoV-2 or SARS-CoV virus skin infection, comprising the steps of:
- taking a sample of skin tissue and isolating skin cells, culturing the skin cells of said skin tissue under appropriate conditions able to provide a keratinocyte cells layer,
- applying a treatment onto said keratinocyte cells layer,
- measuring by quantitative RT-PCR the gene expression levels of at least Androgen receptors (AR), Angiotensin Converting Enzyme 2 (ACE2) receptors and host cell protease (TMPRSS2),
- comparing the values with standard expression levels of same gene target receptors previously determined without SARS treatment
- noting the modulation of AR and/or ACE2 and/or TMPRSS2 gene expression when AR and/or ACE2 and/or TMPRSS2 gene expression level with product application is increased more than two (2) folds or decreased more than 20% in comparison with gene expression level without product application and formulate a conclusion.

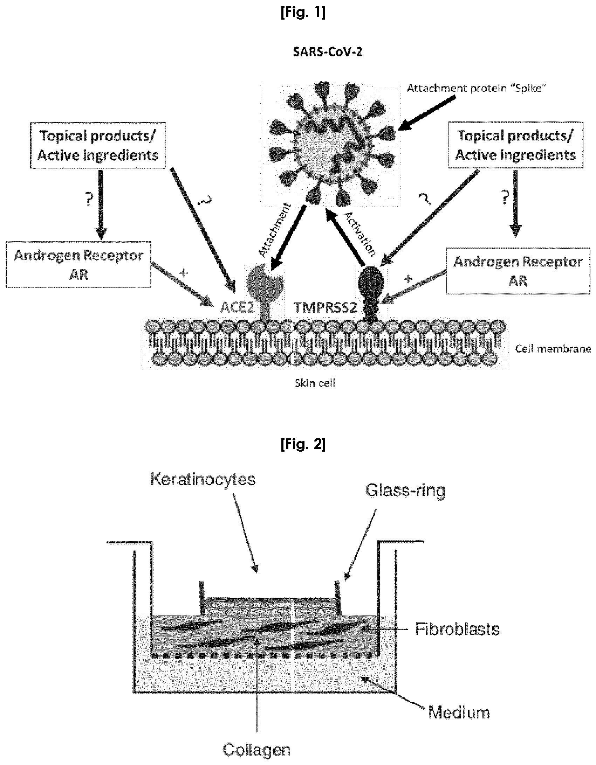

Grounds of invention rely in that skin is a potential door entry of SARS-coronavirus and more preferably the SARS-CoV-2 in the body and thus topical products or active ingredients can prevent skin infection of SARS-coronaviruses and more preferably of SARS-CoV-2. Indeed, damaged skin with compromised barrier integrity facilitates the entry of the virus in the body. However, the virus needs to have key receptors expressed in the target cell to enter the cell. The molecular mechanisms by which topical products or active ingredients prevent skin infection of SARS-CoV-2 are summarized in the scheme presented in Figure 1.

In addition to that, as mentioned above, ACE2 receptors are the door through which the virus enters into cells (Verdecchia et al., 2020). The efficiency of ACE2 usage was found to be a key determinant of SARS-CoV-2 transmissibility. SARS-CoV-2 employs the cellular serine protease TMPRSS2 for S protein priming. Priming of coronavirus S proteins by host cell proteases is essential for viral entry into cells (Wambier et al., 2020).

Androgen receptor (AR) activity is required for the transcription of TMPRSS2 gene as no other regulatory element of the TMPRSS2 promoter has been described in humans to date. ACE2 that is implicated in SARS-CoV-2 viral anchoring to the cell surface, is also affected by androgens, with higher activity found in males.

Male susceptibility to the development of severe COVID-19 symptoms may be further enhanced by X-linked inheritance, since both the androgen receptor gene and the ACE2 genes are located on the chromosome X.

More particularly, the invention relies on SARS-coronavirus and preferably SARS-CoV-2 target expression receptors in skin cells for the first time. In particular, as shown in examples, inventors have shown for the first time that at least 3 of key genes involved in SARS-CoV and SARS-CoV-2 cell entry are expressed in keratinocytes and fibroblasts cells originated from human skin as well as in 3-D reconstructed human epidermis. More particularly, the keratinocytes and the fibroblasts cells in both human skin and 3-D reconstructed human epidermis (also denoted here after as 3-D skin model or as 3-D model) are able to express or modulate the expression of at least Androgen receptors (AR), Angiotensin Converting Enzyme 2 (ACE2) receptors and on S protein priming by host cell protease TMPRSS2.

Preferably, the sample of skin tissue is taken by skin biopsy sample is for example a scrape biopsy comprising a strip or square of skin or is a punch biopsy sample of approximately 4 mm in area and 2 mm in depth, more preferably from human. It will be appreciated that the autologous dermally derived fibroblast and epidermally derived keratinocytes are prepared from the skin biopsy sample according to the well-known methods by one skilled in the art.

In a one embodiment, the instant invention provides an assay to measure target receptors mentioned previously genes expression modulation. This assay comprises the step of direct infection of the skin, either 3D reconstructed human epidermis or excised human skin.

For this purpose, the different evaluation assays included in the scope of invention, are achieved individually and/or in combination selectively or all together:
1- Evaluation of the effect of topical products or active ingredients or botanical extracts on AR regulation in human skin cells or 3D skin tissue, comprising the steps of:
   - measuring by quantitative real time RT-PCR the AR gene expression level with product application in comparison with AR gene expression level without product application (he below defined as "basal level"),
   - noting the modulation of AR gene expression when AR gene expression level with product application is increased more than two (2) folds or decreased more than 20% in comparison with AR gene expression level without product application.

Accordingly, when the gene level expression is at least 20% below the basal level, thus the topical product downregulates expression of androgen receptor (AR). It can be concluded that the topical product accelerates the wound healing by blockade of androgen action via androgen receptor, because it is known that blockade of AR activation accelerates wound healing. On the other hand, downregulation of AR may result in negative action on the activation of both ACE2 and TMPRSS2.

2- Evaluation of the effect of topical products or active ingredients or botanical extracts on ACE2 regulation in skin cells or 3D skin tissue comprising the steps of:
- a) measuring by quantitative real time RT-PCR the ACE2 gene expression level with product application in comparison with AR gene expression level without product application (he below defined as "basal level") or
- a') measuring by the SARS-CoV-2 Inhibitor Screening Kit based on ELISA assay, the binding of the Spike protein from SARS-CoV-2 to its human receptor ACE2 with an active ingredient (in solution) in comparison without active ingredient,
- noting the modulation of ACE2 gene expression when ACE2 gene expression level with product application is expression level is increased more than two (2) folds or decreased more than 20% in comparison with ACE2 gene expression level without product application.

Accordingly, when the gene level expression is at least 20% below the basal level, thus the topical product downregulates expression of ACE2. It can be concluded that the topical product prevents the entry of the SARS-CoV-2 into skin cells, by decreasing the interaction of ACE2 with spike protein that facilitates viral entry into target cells. On the other hand, results from ELISA assay can confirm the inhibitory effect of active ingredients on the binding of spike protein to ACE2.

3- Evaluation of the effect of topical products or active ingredients on TMPRSS2 regulation in skin cells or tissue comprising the steps of:
- measuring by quantitative real time RT-PCR the TMPRSS2 gene expression level with product application in comparison with TMPRSS2 gene expression level without product application (he below defined as "basal level"),
- noting the modulation of TMPRSS2 gene expression when TMPRSS2 gene expression level with product application is expression level is increased more than two (2) folds or decreased more than 20% in comparison with TMPRSS2 gene expression level without product application

Accordingly, when the gene level expression is at least 20% below the basal level, thus the topical product downregulates expression of TMPRSS2. It can be concluded that the topical product prevents the entry of the SARS-CoV-2 into skin cells, by decreasing the interaction of TMPRSS2 with spike protein that facilitates viral entry into target cells.

Most of topical products or topical drugs can be evaluated by the assays described above. In particular topical formulated products such as cream, lotion, oil, semi-liquid formulation. Among topical drugs assessed by the assays, all drugs formulated in a topical medium are in the scope of the invention. As illustrating examples, drugs such as all the actives acting on Androgen receptor (AR) mechanism of action.

Most of applicable 3-D skin models in the context of the present invention are Current 3-D skin models use either: (i) keratinocytes and fibroblasts (derived from the epidermis or dermis respectively) from excess skin from plastic surgery patients or (ii) immortalized cell lines. In a preferred embodiment, the 3D-skin model is a 3D reconstructed human epidermis.

A particularly interesting of *in vitro* model applicable in the instant invention, is an *in vitro* reconstructed skin which consists of a dermal equivalent with human fibroblasts overlaid by a stratified, well differentiated epidermis derived from normal human keratinocytes cultured on an inert polycarbonate filter.

Another applicable *in vitro* skin model for the present invention is a reconstructed human epidermis (RHE) model. With this model, authors of article "Skin concentration of topically applied substances in reconstructed human epidermis (RHE) compared with human skin using in vivo confocal Raman microscopy "(Fleischli FD., Morf F., Adlhart C., Chimia, 69, 147-151, 2015) have compared penetration of substances with this model and human skin.

Figures described below and in the appendices are illustrative of the instant invention:
Figure 1 illustrates the Molecular mechanism by which topical products/active ingredients may prevent skin infection of SARS-CoV-2.
Figure 2 shows the transversal representation of skin cells layers (keratinocytes and fibroblasts) in a 3-D skin model.
Figure 3 shows measurement of basal expression level of AR, ACE2 and TMPRSS2 in primary culture of human keratinocytes cultivated for 24 hours. mRNA expression was measured by quantitative real time RT-PCR.
Figure 4 shows measurement of basal expression level of AR, ACE2 and TMPRSS2 in primary culture of human keratinocytes cultivated for 48 hours. mRNA expression was measured by quantitative real time RT-PCR.
Figure 5 shows measurement of basal expression level of AR, ACE2 and TMPRSS2 in primary culture of human fibroblasts cultivated for 24 hours. mRNA expression was measured by quantitative real time RT-PCR.
Figure 6 shows measurement of basal expression level of AR, ACE2 and TMPRSS2 in 3D reconstructed human epidermis from Provider 1. mRNA expression was measured by quantitative real time RT-PCR.
Figure 7 shows measurement of basal expression level of AR, ACE2 and TMPRSS2 in 3D reconstructed human epidermis from Provider 2. mRNA expression was measured by quantitative real time RT-PCR.

The following examples illustrate the invention but not be considered as a limitation of the scope of the instant invention.

### EXAMPLE 1: Measurement of basal expression level of AR, ACE2 and TMPRSS2 in primary culture of human keratinocytes cultivated for 24 hours.

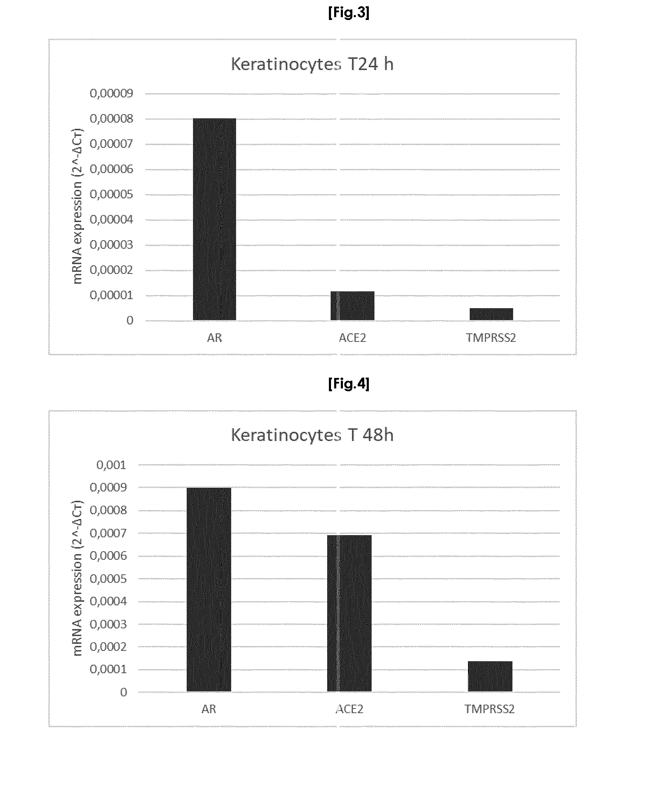

The present example provides the demonstration that AR, ACE2 and TMPRSS2 in primary culture of human keratinocytes (constitutive cells of skin epidermis) cultivated for 24 hours. The basal expression level of mRNA expression was measured by quantitative real time RT-PCR as shown in figure 3 and all the 3 receptors genes are expressed.

The quantitative real time RT-PCR is a gold standard method well known from the skilled artisan and enables reliable detection and measurement of products generated during each cycle of PCR process.

### EXAMPLE 2: Measurement of basal expression level of AR, ACE2 and TMPRSS2 in primary culture of human keratinocytes cultivated for 48 hours.

The present example provides the demonstration that AR, ACE2 and TMPRSS2 receptors genes are expressed in primary culture of human keratinocytes cultivated for 48 hours. The basal expression level of mRNA expression was measured by quantitative real time RT-PCR as shown in figure 4. The 3 receptors genes are expressed in a higher proportion than at 24h as shown in example 1.

### EXAMPLE 3: Measurement of basal expression level of AR, ACE2 and TMPRSS2 in primary culture of human fibroblasts cultivated for 24 hours.

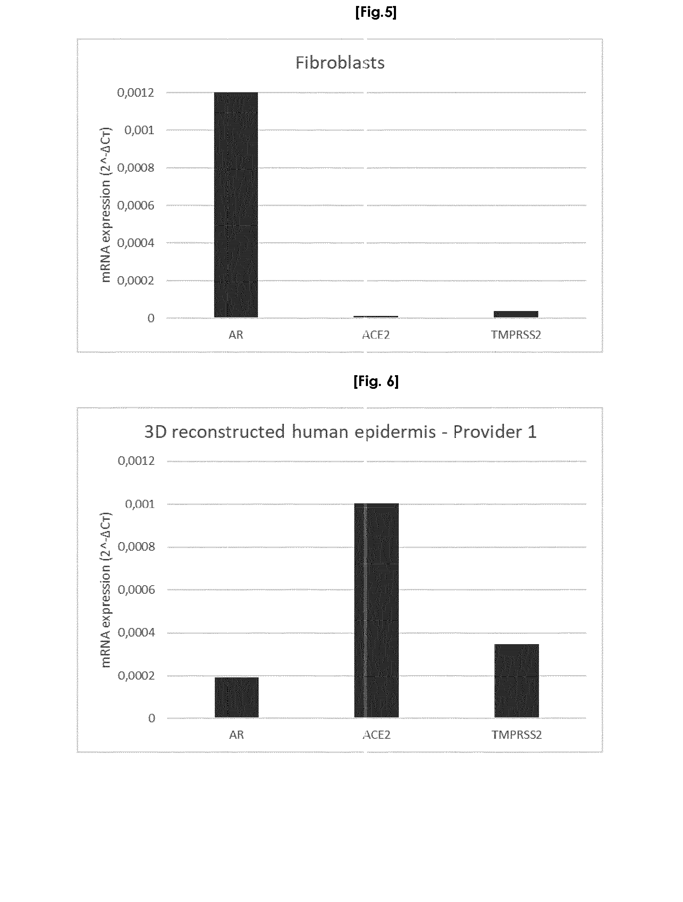

The present example is demonstrating that AR, ACE2 and TMPRSS2 receptors genes are expressed in primary culture of human fibroblasts cultivated for 24 hours. The basal expression level of mRNA expression was measured by quantitative real time RT-PCR as shown in figure 5. As shown, androgen receptor (AR) is expressed in a large proportion but it is noticed that ACE-2 and TMPRSS2 are also expressed.

### EXAMPLE 4: Measurement of basal expression level of AR, ACE2 and TMPRSS2 in 3D reconstructed human epidermis (from Provider 1).

The present example clearly demonstrates that AR, ACE2 and TMPRSS2 receptors genes are expressed in 3D reconstructed human epidermis.

The basal expression level of mRNA expression was measured by quantitative real time RT-PCR as shown in figure 6. As shown, the 3 receptors genes are expressed with a large proportion for ACE-2. It is noticeable that TMPRSS2 receptor gene is expressed at a higher level.

### EXAMPLE 5: Measurement of basal expression level of AR, ACE2 and TMPRSS2 in 3D reconstructed human epidermis (from Provider 2).

The present example clearly demonstrates that AR, ACE2 and TMPRSS2 receptors genes are expressed in 3D reconstructed human epidermis.

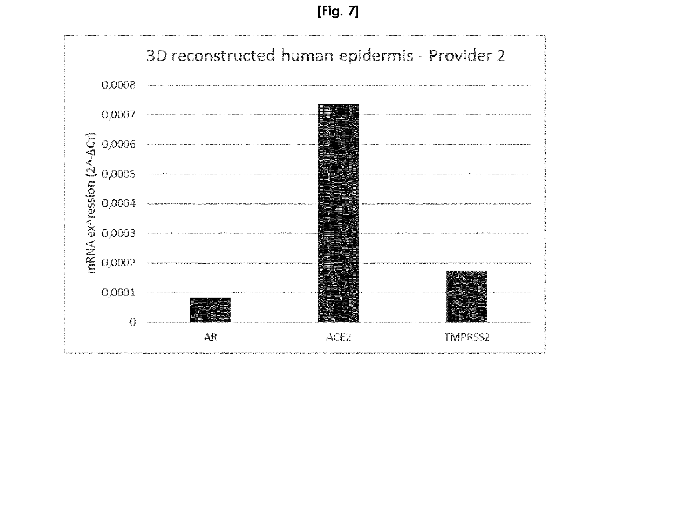

The basal expression level of mRNA expression was measured by quantitative real time RT-PCR as shown in figure 7. Similar results as shown in example 4 and figure 6 are measured. These results are showing the robustness of the data and thus confirming the scientific approach.

All together, these examples are demonstrating that for the first time, at least 3 of key genes involved in SARS-CoV and SARS-CoV-2 cell entry, are expressed in keratinocytes and fibroblasts cells originated from human skin as well as in 3-D reconstructed human epidermis(also denoted here after as 3-D skin model or as 3-D model).

More particularly, the keratinocytes and the fibroblasts cells in both human skin and 3-D reconstructed human epidermis are able to express and/or modulate the expression of at least Androgen receptors (AR), Angiotensin Converting Enzyme 2 (ACE2) receptors and on S protein priming by host cell protease TMPRSS2.

## Claims

1. An *in vitro* assay for assessing treatment of SARS-Coronavirus skin infection, comprising the steps of:
- taking a sample of skin tissue and isolating skin cells, culturing the skin cells of said skin tissue under appropriate conditions able to provide a keratinocyte cells layer,
- applying a treatment onto said keratinocyte cells layer,
- measuring by quantitative RT-PCR the gene expression levels of at least Androgen receptors (AR), Angiotensin Converting Enzyme 2 (ACE2) receptors and host cell protease (TMPRSS2),
- comparing the values with standard expression levels of same gene target receptors previously determined without SARS treatment,
- noting the modulation of AR and/or ACE2 and/or TMPRSS2 gene expression when AR and/or ACE2 and/or TMPRSS2 gene expression level with product application is increased more than two (2) folds or decreased more than 20% in comparison with gene expression level without product application.

2. *In vitro* Assay according to claim 1, wherein SARS-coronavirus is SARS-CoV-2 virus or SARS-CoV virus.

## Patentansprüche

1. *In-vitro-Assay* zur Bewertung einer Behandlung einer SARS-Coronavirus-Hautinfektion, umfassend die Schritte, die darin bestehen,:
- eine Probe von Hautgewebe zu entnehmen und Hautzellen zu isolieren, die Hautzellen des genannten Hautgewebes unter geeigneten Bedingungen zu kultivieren, die geeignet sind, eine Schicht von Keratinozytenzellen bereitzustellen,
- eine Behandlung auf die genannte Schicht von Keratinozytenzellen aufzubringen,
- mittels quantitativer RT-PCR die Genexpressionsniveaus von mindestens dem Androgenrezeptor (AR), dem ACE2-Rezeptor (Angiotensin-Converting-Enzym 2) und der Wirtszellprotease (TMPRSS2) zu messen,
- die Werte mit Standard-Expressionsniveaus derselben Gen-Zielrezeptoren zu vergleichen, die zuvor in Abwesenheit einer Behandlung gegen SARS bestimmt wurden,
- die Modulation der Genexpression von AR und/oder ACE2 und/oder TMPRSS2 festzustellen, wenn das Genexpressionsniveau von AR und/oder ACE2 und/oder TMPRSS2 in Gegenwart der Produktapplikation im Vergleich zum Genexpressionsniveau in Abwesenheit der Produktapplikation um mehr als das Zweifache (2-fach) erhöht oder um mehr als 20 % verringert ist.

2. *In-vitro-Assay* nach Anspruch 1, wobei das SARS-Coronavirus das Virus SARS-CoV-2 oder das Virus SARS-CoV ist.

## Revendications

1. Essai *in vitro* permettant d'évaluer un traitement d'une infection cutanée à SARS-coronavirus, comprenant les étapes de :
- prélever un échantillon de tissu cutané et isoler des cellules cutanées, mettre en culture les cellules cutanées dudit tissu cutané dans des conditions appropriées aptes à fournir une couche de cellules kératinocytaires,
- appliquer un traitement sur ladite couche de cellules kératinocytaires,
- mesurer, par RT-PCR quantitative, les niveaux d'expression génique d'au moins le récepteur aux androgènes (AR), le récepteur Enzyme de Conversion de l'Angiotensine 2 (ACE2) et la protéase de la cellule hôte (TMPRSS2),
- comparer les valeurs à des niveaux d'expression standards des mêmes cibles géniques, préalablement déterminés en l'absence de traitement contre le SARS,
- noter la modulation de l'expression génique de AR et/ou de ACE2 et/ou de TMPRSS2 lorsque le niveau d'expression génique de AR et/ou de ACE2 et/ou de TMPRSS2 en présence de l'application du produit est augmenté de plus de deux (2) fois ou diminué de plus de 20 % par rapport au niveau d'expression génique en l'absence d'application du produit.

2. Essai *in vitro* selon la revendication 1, **caractérisé en ce que** le SARS-coronavirus est le virus SARS-CoV-2 ou le virus SARS-CoV.
